# EUROPEAN PATENT SPECIFICATION

(11) **EP 0 708 794 B1**
(45) Date of publication and mention of the grant of the patent: **22.09.1999**
(21) Application number: 94923920.6
(22) Date of filing: 14.07.1994
(51) Int. Cl.: C08G 65/10, C08G 65/04, C08G 65/20

(54) **POLYMERIZATION OF CYCLIC ETHERS USING HETEROGENEOUS CATALYSTS**
POLYMERISATIONSKATALYSATOR FÜR POLYETHER
POLYMERISATION D'ETHERS CYCLIQUES A L'AIDE DE CATALYSEURS HETEROGENES

(30) Priority: 16.07.1993 US 93119
(43) Date of publication of application: 01.05.1996
(60) Divisional application: 96202116.8
(73) Proprietor: E.I. DU PONT DE NEMOURS AND COMPANY, Wilmington Delaware 19898 (US)
(72) Inventor: DRYSDALE, Neville, Everton, Newark, DE 19702-1026 (US); HERRON, Norman, Newark, DE 19711 (US)
(74) Representative: Jones, Alan John
(86) International application number: US9407590
(87) International publication number: WO9502625

(56) References cited:
- EP-A- 0 004 536
- WO-A-94/09055
- DE-A- 1 915 117
- DE-A- 3 606 479
- FR-A- 2 212 364
- US-A- 3 842 019
- US-A- 5 102 849
- DATABASE WPI Derwent Publications Ltd., London, GB; AN 94-068607 & JP,A,6 016 805 (DAICEL CHEM IND) 25 January 1994
- DATABASE WPI Derwent Publications Ltd., London, GB; AN 85-181197(30) & JP,A,60 109 584 (ASAHI CHEM IND KK) 15 June 1985
- DATABASE WPI Derwent Publications Ltd., London, GB; AN 87-353218(50) & JP,A,62 257 931 (ASAHI CHEMICAL IND KK) 10 November 1987

## Description

### FIELD OF THE INVENTION

Polymerization of cyclic ethers to polyethers is catalyzed by heterogeneous catalysts which contain selected metal cations. The metal cation may be the charge balancing counterion of a zeolite structure.

### TECHNICAL BACKGROUND

Cyclic ethers are polymerized by various means to give products of widespread utility. For instance, ethylene oxide is polymerized to polyethylene oxide which is useful for (in lower molecular weight grades) ceramics, cosmetics, lubricants, polyurethanes, (and in higher molecular weight grades), packaging film, denture adhesives, lubricants, and flocculation, and tetrahydrofuran (THF) is polymerized to poly(tetramethylene ether) glycol which is useful in the preparation of Spandex fibers, polyurethane resins useful in elastomeric parts, and thermoplastic elastomers useful for molding various mechanical parts. Therefore, improved methods of making these polymers are sought.

U.S. Patent 4,303,782 describes the use of zeolites to catalyze the polymerization of tetrahydrofuran. These polymerization appear to proceed very slowly.

U.S. Patent 3,842,019 describes the polymerization of oxiranes and other small ring compounds by a presumed cationic mechanism, using as the catalyst the decomposition products of metal perfluoroalkyl-sulfonates. These catalysts are described as "latent", that is no reaction occurs until the metal salt is decomposed. The reactions reported are relatively slow, even at elevated temperatures.

U.S. Patents 5,084,586 and 5,124,417 describe the cationic polymerization of various monomers, including cyclic ethers, using onium cations, whose corresponding anions are fluoroalkylsulfatometallates. Onium ion catalyzed cationic polymerizations are well known, and there is no mention in these patents of the use of metal salts not containing onium ions, such as metal triflates, as catalysts for the polymerization of cyclic ethers.

Japanese Patent Application 51-82397 describes the polymerization of tetrahydrofuran using a combination of fluorosulfonic acid and a carboxylic acid as catalysts. No mention is made of metal salts, such a metal triflates as catalysts.

J. S. Hrkach, et al., Macromolecules, vol. 23, p. 4042-4046 (1990) describe the polymerization of tetrahydrofuran using trimethylsilyl trifluoromethanesulfonate (herein triflate) as the initiator. No mention is made of any other triflates as catalysts for this polymerization.

S. L. Borkowsky, et al., Organometal., vol. 10, p. 1268-1274 (1991) report that certain zirconium complexes can initiate the polymerization of tetrahydrofuran. No mention is made of zirconium perfluoroalkyl-sulfonates, or of copolymers.

T. Misaki, et al., Nippon Kagaku Kaishi, p. 168-174 (1973) report on the polymerization of THF using a combination of metal aceylacetonates and acetyl chloride. None of the metal compounds herein disclosed as catalysts are mentioned.

Japanese Patent Application 51-82397 describes the polymerization of tetrahydrofuran using a combination of fluorosulfonic acid and a carboxylic acid as catalysts. No mention is made of metal salts as catalysts.

### SUMMARY OF THE INVENTION

This invention concerns a process for the polymerization of cyclic ethers, comprising, contacting one or more oxetanes, tetrahydrofurans, oxepanes, 1,3-dioxolanes, or 1,3,5-trioxanes; with a zeolite which contains a metal cation selected from the group consisting of scandium, yttrium, the rare earth metals, titanium, zirconium, hafnium, vanadium, niobium, chromium, molybdenum, tantalum, tungsten, rhenium, iron, ruthenium, osmium, rhodium, iridium, palladium, copper, platinum, silver, gold, zinc, cadmium, mercury, aluminum, gallium, indium, thulium, germanium, tin, lead, arsenic, antimony and bismuth; and an accelerator selected from the group consisting of carboxylic anhydrides, acyl halides and carboxylic acids whose pKa in water is less than about 6.

### DETAILS OF THE INVENTION

In the polymerization processes described herein one or more cyclic ethers, oxetanes, tetrahydrofurans, oxepanes, 1,3-dioxolanes, or 1,3,5-trioxanes are polymerized to form a polyether. Oxetane is given its common meaning, a saturated four membered ring containing 3 carbon atoms and one oxygen atom. The term 1,3-dioxolane means a saturated 5 membered ring which contains two oxygen atoms separated by 1 carbon atom. The term 1,3,5-trioxane means a six membered ring containing 3 oxygen atoms in which the oxygen atoms and carbons atoms are alternated.

The terms oxetane, oxepane, 1,3-dioxolane, 1,3,5-trioxane, and tetrahydrofuran include compounds containing those ring systems which are substituted with hydrocarbyl or hydrocarbylene groups containing 1 to 20 carbon atoms. The hydrocarbylene groups form carbocyclic rings, which include bicyclic, tricyclic, etc. systems. By a hydrocarbylene group herein is meant a divalent radical containing carbon and hydrogen which is part of a carbocyclic ring.

Preferred cyclic ethers have the formula 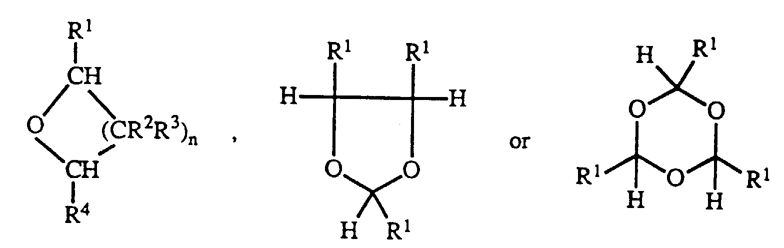 wherein n is 2 or 4, and each R¹, R², R³ and R⁴ is independently hydrogen or hydrocarbyl containing 1 to 20 carbon atoms. Some cyclic ethers polymerize to give repeat units of the formula -[CHR¹(CR²R³)ₙCHR⁴O]-. In a more preferred cyclic ether all of R¹, R², R³ and R⁴ are hydrogen. In another more preferred cyclic ether where n=2, R¹, one of R², both of R³ and R⁴ are hydrogen, and the remaining R² is alkyl containing 1-4 carbon atoms, especially preferably the remaining R² is methyl. By hydrocarbyl herein is meant a univalent radical containing carbon and hydrogen.

The polymerization is carried out in the presence of an accelerator (sometimes also called a co-catalyst). Suitable accelerators are carboxylic anhydrides, acyl halides and carboxylic acids whose pKa is less than about 6 in water.

By a carboxylic anhydride is meant a compound containing the grouping -C(O)O(O)C-, wherein the free valencies are to other carbon atoms. A preferred carboxylic anhydride is an anhydride of an alkyl carboxylic acid or a halogen substituted alkyl carboxylic acid, and particularly preferred anhydrides are acetic anhydride and trifluoroacetic anhydride.

By an acyl halide is meant a compound containing the grouping -C(O)X, where X is chlorine or bromine and the free valence it to another carbon atom. In preferred acyl halides X is chlorine. Preferred acyl halides are alkyl acyl halides, and especially preferred are acetyl halides, more preferably acetyl chloride.

By a carboxylic acid is meant a compound containing the grouping -C(O)OH, wherein the free valence is to another carbon atom. Preferred carboxylic acids have a pKa of less than 5 in water. Useful carboxylic acids include, but are not limited to formic, acetic, trifluoroacetic, chloroacetic, benzoic, trichloroacetic, p-nitrobenzoic, butyric, and naphthoic acids. Preferred carboxylic acids are trifluoroacetic, formic, acetic, cyanoacetic, nitropropionic, nitrobenzoic, acrylic and methacrylic. These and other acids that themselves don't cause polymerization of the cyclic ethers are also believed to be accelerators, especially if their pKa in water is about 6 or less.

When carboxylic anhydride is present one half or more of the end groups are carboxylic esters. As is known to the artisan, these may be hydrolyzed to hydroxyl groups by reaction with water, preferably in the presence of a catalyst, such as a strong acid (sulfuric acid for instance) or a strong base (such as NaOH). The proportion of acetate ends increases the longer the polymerization is allowed to proceed. Although the polymeric diol is often the desired product (it can be used to make other polymers, such as polyurethanes and polyesters), the half ester or diester is also useful, as in relatively low molecular weight polymers which can be used as solvents.

When acyl halides are used as the accelerator, the end groups are usually ester on one end, and the halide, X, on the other. Thus the complete formula for such a polymer could be X-[CHR¹(CR²R³)ₙCHR⁴O]-C(O)Y, where Y is the group to which the acyl group of the acyl halide was bound. Such polymers are useful as intermediates for the preparation of polymers containing different functional groups. For example, the ester may be hydrolyzed to a hydroxyl group, and the halide may be reacted to form another functional group such as nitrile. If a bis(acyl halide), X(O)CYC(O)X, is used as the accelerator, the product of the polymerization will be a polyether with halide (X) end groups which contains two internal ester groups, and may have the formula X-[CHR¹(CR²R³)ₙCHR⁴O]-C(O)YC(O)-[OCHR¹(CR²R³)ₙCHR⁴]-X. Useful bis(acyl halides) include adipoyl chloride, terephthaloyl chloride, and diglycolyl chloride [Cl(O)CCH₂OCH₂C(O)Cl].

When a carboxylic acid is used as the accelerator, the end groups are usually mostly ester. Thus the complete formula for such a polymer could be Y-C(O)-O-[CHR¹(CR²R³)ₙCHR⁴O]-C(O)Y, where Y is the group to which the acyl group of the carboxylic acid was bound. The ester group may be hydrolyzed as described above in the paragraph describing the products when a carboxylic anhydride is used as the accelerator.

The polymerization may be run at a temperature of about -80°C to about 130°C. If this temperature is above the boiling point of the cyclic ether monomer, a pressure vessel may be used. A preferred temperature is ambient to the boiling point of the monomer or 110°C, whichever is lower. An inert solvent such as di-n-butyl ether, diethyl ether or toluene may be used, but it is preferred if solvents are not present. Protic compounds such as water, methanol and ethanol should preferably not be present, and it is convenient to exclude them by drying the starting materials and keeping the process under an inert dry gas such as nitrogen or dry air. As in most chemical processes, the ingredients should be mixed. Continued agitation is preferred to assure contact of the process liquids with the heterogeneous catalyst, and to avoid overheating. The polymerization is mildly exothermic. If the polymerization temperature goes up appreciably, refluxing of the monomer may be used to help cool the process.

The polymerization may be run in a variety of methods, such as batch, semi-continous, or continuous. While the heterogeneous catalyst may be recovered each time, as by filtration, and reused, in another embodiment the catalyst is fixed in place and the polymerization mass circulated or agitated so that uniform contact with the catalyst surface is obtained. In this way, the catalyst may be used for long periods in a continuous process, or for many batches in a batch polymerization, without the need to recover the catalyst. Contact time of the liquid reaction mass with the catalyst will depend on many factors, such as the catalytic metal used, its concentration on the catalyst, the temperature, cyclic ether being polymerized, etc., but will usually be in the range of a few minutes to a few hours.

The catalysts used herein contain selected metal cations. Preferred metal cations are those of scandium, yttrium, the rare earth metals, titanium, zirconium, hafnium, vanadium, niobium, tantalum, chromium, molybdenum, tungsten, rhenium, iron, ruthenium, palladium, copper, gold, zinc, tin and bismuth. More preferred metals are yttrium, the rare earth metals, scandium and zirconium. Especially preferred metals are yttrium, ytterbium, dysprosium, erbium, neodymium, lanthanum, scandium and zirconium. Another preferred metal is "mischmetall" (sometimes also called "didymium"), which is a mixture of rare earth metals as obtained from the ore. By rare earths herein is meant lanthanum, cerium, praeseodymium, neodymium, promethium, samarium, europium, gadolinium, terbium, dysprosium, holmium, erbium, thulium, ytterbium, and lutetium.

The catalysts used herein include zeolites in which some of the metal cations present are the cations listed above. The anions of such cations are not critical, and may be those normally found in zeolites. The zeolites containing the appropriate metal cations can be made by the ion exchange of cations in known zeolites. Such ion exchange processes are know to the artisan, see for instance D. W. Breck in Zeolite Molecular Sieves, R.E. Krieger Publishing Co., Malabar, Florida, 1984 and Examples 7, 9, 12, 14, 16, 18, and 20. It is preferred if at least 0.5 atom percent of the metals and metalloids in the zeolite are one of the useful "catalytic" metals, more preferably at least 5 atom percent. It has been found that the zeolite catalysts usually yield polyethers with bimodal molecular weight distributions.

In the polymerization processes disclosed herein the heterogeneous catalyst may be recovered and reused. It may be recovered from the processes by filtration, and if desired, drying. The recovered catalyst may be used again in the polymerization processes.

The above processes may be carried out as batch, semibatch or continuous processes and continuous type processes are preferred.

In the Examples, the following abbreviations are used:
ACA - acetic anhydride
DETM - diethyl 2-[3-(triethoxysilyl)propyl)]malonate
GPC - gel permeation chromatography
Mn - number average molecular weight
Mw - weight average molecular weight
PD - polydispersity, Mw/Mn
PS - polystyrene
SS - stainless steel
STD - standard
THF - tetrahydrofuran

### EXAMPLE 1

### Preparation of La loaded zeolite HY for THF polymerization

10 g of zeolite LZY-82 (NH₄⁺ ion form of zeolite Y) was slurried into 1 liter distilled water and the pH was adjusted to 4 with nitric acid. 1.56 g lanthanum nitrate hexahydrate (calculated to give a final product having ∼5wt% La) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min):
Room temperature to 500°C at 12°C/min.
Hold at 500°C for 2hours
Cool to room temperature over lhour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk La₂O₃ phase.

### EXAMPLE 2

### Polymerization of THF with Lanthanum Zeolite and Acetic Anhydride

In a dry box, the lanthanum zeolite of Example 1 (2.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight the resulting material was diluted with THF (50 mL) and filtered. The resulting filtrate was concentrated at reduced pressure. The vicous liquid was dissolve in THF (50 mL) and dried over anhydrous NaHCO₃, filtered concentrated at reduced pressure and then dried under vacuum. Polymer yield: 3.86 g. GPC analysis: Mn = 969, Mw = 5970, PD = 6.17 (PS STD., bimodal distribution).

### EXAMPLE 3

### Preparation of 5% Y loaded zeolite HY for THF polymerization

10 g of zeolite LZY-82 (NH₄⁺ ion form of zeolite Y) was slurried into 1 liter distilled water and the pH was adjusted to 4 with nitric acid. 2.20 g yttrium nitrate pentahydrate (calculated to give a final product having -5 wt% Y) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min):
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk Y₂O₃ phase.

### EXAMPLE 4

### Polymerization of THF with 5 Yttrium Zeolite and Acetic Anhydride

In a dry box, the yttrium zeolite of Example 3 (5.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight ether (50 mL was added to the resulting polymerized solution. After filtering, ether (50 mL) and water (25 mL) were added and the organic phase separated, concentrated at reduced pressure and then dried under vacuum. Polymer yield: 2.21 g. GPC analysis: Mn = 840, Mw = 4600, PD = 5.48 (PS STD., bimodal distribution).

### EXAMPLE 5

### Polymerization of THF with 5% Yttrium Zeolite HY and Acetic Anhydride

In a dry box, the 5% yttrium zeolite HY of Example 3 (10.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight ether (50 mL) was added to the resulting polymerized solution. After filtering, ether (50 mL) and water (25 mL) were added and the organic phase separated, concentrated at reduced pressure and then dried under vacuum. Polymer yield: 7.29 g. GPC analysis: Mn = 1350, Mw = 14800, PD = 10.94 (PS STD., bimodal distribution).

### EXAMPLE 6

### Preparation of 10 wt% Y loaded zeolite HY for THF polymerization

50 g of zeolite LZY-82 (NH₄⁺ ion form of zeolite Y) was slurried into 1 liter distilled water and the pH was adjusted to 4 with nitric acid. 22.0 g yttrium nitrate pentahydrate (calculated to give a final product having ∼10 wt% Y) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min):
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk Y₂O₃ phase.

### EXAMPLE 7

### Polymerization of THF with 10 wt% Yttrium Zeolite HY and Acetic Anhydride

In a dry box, the 10% yttrium zeolite HY of Example 6 (10.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After 4 hrs. ether (50 mL) was added to the resulting polymerized solution. After filtering, the organic phase was washed with 5% NaOH (10 mL), separated concentrated at reduced pressure then dried under vacuum. Polymer yield: 6.11 g. GPC analysis:
Mn = 690, Mw = 4120, PD = 10.94 (PS STD., bimodal distribution).

### EXAMPLE 8

### Preparation of Y loaded zeolite mordenite for THF polymerization

10 g of zeolite H-mordenite was slurried into 1 liter distilled water and the pH was adjusted to 4 with nitric acid. 2.20 g yttrium nitrate pentahydrate (calculated to give a final product having ∼5 wt% Y) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min):
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk Y₂O₃ phase.

### EXAMPLE 9

### Polymerization of THF with Yttrium Mordenite and Acetic Anhydride

In a dry box, the yttrium mordenite of Example 8 (5.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight ether (50 mL) was added to the resulting polymerized solution. After filtering, ether (50 mL) and water (25 mL) were added and the organic phase separated, concentrated at reduced pressure and then dried under vacuum. Polymer yield: 1.43 g. GPC analysis: Mn = 6020, Mw = 15500, PD = 2.58 (PS STD.).

### EXAMPLE 10

### Preparation of ∼10 wt% Didymium loaded zeolite HY for THF polymerization

50 g of zeolite LZY-82 (NH₄⁺ ion form of zeolite Y) was slurried into 1 liter distilled water and the pH was adjusted to 4 with nitric acid. 10 g didymium chloride was added and the slurry stirred and warmed for 1 hour. The zeolite was recovered by filtration washed with 1 liter distilled water and then re-slurried into 1 liter fresh distilled water. Another 10 g didymium chloride was added and the pH adjusted to 4 with nitric acid and this slurry was stirred for 1 hour. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min):
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. The powder was then pressed to 10 tons and the resultant cake sieved through 10-20 mesh to give granulated catalyst.

### EXAMPLE 11

### Polymerization of THF with 10% Didymium Zeolite HY and Acetic Anhydride

In a dry box, the 10% Didymium zeolite HY of Example 10 (10.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight the polymerized solution was fitered, then ether (25 mL), THF (25 mL) and water (25 mL) were added the organic phase separated concentrated at reduced pressure then dried under vacuum. Polymer yield: 7.56 g. GPC analysis:
Mn = 1010, Mw = 7150, PD = 7.08 (PS STD., bimodal distribution).

### EXAMPLE 12

### Preparation of 5 wt% Sc loaded zeolite HY for THF polymerization

5 g of zeolite LZY-82 (NH₄⁺ ion form of zeolite Y) was slurried into 1 liter distilled water and the pH was adjusted to 4 with nitric acid. 1.44 g scandium chloride (calculated to give a final product having ∼5 wt% Sc) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min):
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk Sc₂O₃ phase.

### EXAMPLE 13

### Polymerization of THF with 5% Scandium Zeolite HY and Acetic Anhydride

In a dry box, the 5% scandium zeolite HY of Example 12 (3.94 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight the polymerized solution was filtered, then ether (25 mL), THF (25 mL) and water (25 mL) were added the organic phase separated concentrated at reduced pressure then dried under vacuum. Polymer yield: 4.48 g. GPC analysis:
Mn = 736, Mw = 7890, PD = 10.72 (PS STD., bimodal distribution).

### EXAMPLE 14

### Preparation of 10 wt% Y on zeolite NaY pellets

12.5 g yttrium nitrate pentahydrate was dissolved in lliter distilled water and the pH adjusted to 4 with nitric acid. 25 g zeolite LZY-52 (NaY) pellets (3 mm dia x 3 mm) was added and the mixture allowed to sit overnight. The pellets were then filtered and washed with lliter distilled water and suction dried. The pellets were then calcined in flowing dry air (flow rate 200 mL/min):
Room temperature to 500°C at 12°C/min.
Hold at 500°C for 2 hours.
Cool to room temperature over 1 hour and collect white powder.

The cool pellets were quickly transferred to a tightly capped sample vial in order to minimize exposure to atmospheric moisture.

### EXAMPLE 15

### Polymerization of THF with 10% Yttrium Triflate on Zeolite NaY Pellets and Acetic Anhydride

In a dry box, the 10% yttrium triflate on zeolite NaY pellets of Example 14 (10.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring for 1 hour THF (50 mL) the polymerized solution then filtered. The filtrate was washed with water (25 mL), then ether (50 mL) was added. The organic phase was separated concentrated at reduced pressure then dried under vacuum. Polymer yield: 0.700 g. GPC analysis: Mn = 18800, Mw = 27100, PD = 1.44 (PS STD.).

### COMPARATIVE EXAMPLE 1

In a dry box, zeolite HY (1.00 g) was added to an oven dry 100 mL RB flask equipped with a stirring bar. The flask was sealed with a rubber septum and removed from the dry box. After the attachment of a nitrogen bleed THF (20.00 mL) was added by syringe. After 45 minutes acetic anhydride (1.00 mL) was added. After filtering off the zeolite catalyst and concentration of the filtrate, no polymer was obtained.

## Claims

1. A process for the polymerization of cyclic ethers, comprising, contacting one or more oxetanes, tetrahydrofurans, oxepanes, 1,3-dioxolanes or 1,3,5-trioxanes; with a zeolite which contains a metal cation selected from the group consisting of scandium, yttrium, the rare earth metals, titanium, zirconium, hafnium, vanadium, niobium, chromium, molybdenum, tantalum, tungsten, rhenium, iron, ruthenium, osmium, rhodium, iridium, palladium, copper, platinum, silver, gold, zinc, cadmium, mercury, aluminum, gallium, indium, thulium, germanium, tin, lead, arsenic, antimony bismuth and mischmetall; and an accelerator selected from the group consisting of carboxylic anhydrides, acyl halides and carboxylic acids whose pKa in water is less than about 6.

2. The process as recited in Claim 1 wherein said cyclic ether is one or more tetrahydrofurans, oxepanes, 1,3-dioxolanes, or 1,3,5-trioxanes.

3. The process as recited in Claim 2 wherein said cyclic ether comprises the formula: 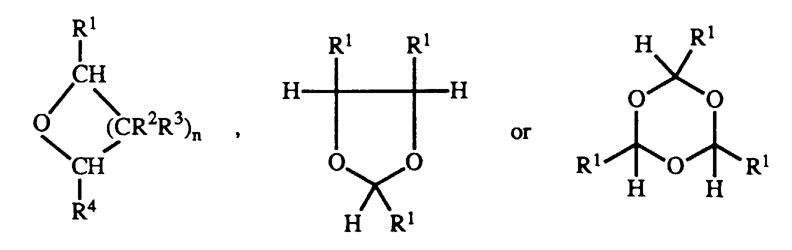 wherein n is 2 or 4, and each R¹, R², R³ and R⁴ is independently hydrogen or hydrocarbyl containing 1 to 20 carbon atoms.

4. The process as recited in Claim 3 wherein all of R¹, R², R³ and R⁴ are hydrogen.

5. The process as recited in Claim 3 wherein n is 2; and wherein R¹, one of R², both of R³ and R⁴ are hydrogen and the remaining R² is alkyl containing 1-4 carbon atoms.

6. The process as recited in Claim 1 wherein said metal cation is scandium, yttrium, the rare earth metals, titanium, zirconium, hafnium, vanadium, niobium, tantalum, chromium, molybdenum, tungsten, rhenium, iron, ruthenium, palladium, copper, gold, zinc, tin bismuth or mischmetall.

7. The process as recited in Claim 6 wherein said metal cation is yttrium, ytterbium, dysprosium, erbium, neodymium, lanthanum, mischmetall, scandium or zirconium.

8. The process as recited in Claim 1 wherein said accelerator is a carboxylic acid whose pKa in water is about 6 or less.

9. The process as recited in Claim 8 wherein said carboxylic acid is selected from the group consisting of formic, acetic, trifluoroacetic, acrylic, methacrylic, cyanoacetic, nitropropionic, and nitrobenzoic.

## Patentansprüche

1. Verfahren zur Polymerisation von zyklischen Ethern, umfassend das Kontaktieren von einem oder mehreren Oxetanen, Tetrahydrofuranen, Oxepanen, 1,3-Dioxolanen oder 1,3,5-Trioxanen; mit einem Zeolith, der ein Metallkation enthält, welches aus der aus Scandium, Yttrium, den Seltenerdmetallen, Titan, Zirkonium, Hafnium, Vanadium, Niob, Chrom, Molybdän, Tantal, Wolfram, Rhenium, Eisen, Ruthenium, Osmium, Rhodium, Iridium, Palladium, Kupfer, Platin, Silber, Gold, Zink, Cadmium, Quecksilber, Aluminium, Gallium, Indium, Thulium, Germanium, Zinn, Blei, Arsen, Antimon, Wismut und Mischmetall bestehenden Gruppe ausgewählt ist; und einem Beschleuniger, der aus der aus Carbonsäureanhydriden, Acylhalogeniden und Carbonsäuren, deren pKa in Wasser weniger als etwa 6 beträgt, bestehenden Gruppe ausgewählt ist.

2. Verfahren nach Anspruch 1, in welchem es sich bei dem zyklischen Ether um ein oder mehrere Tetrahydrofurane, Oxepane, 1,3-Dioxolane oder 1,3,5-Trioxane handelt.

3. Verfahren nach Anspruch 2, in welchem der zyklische Ether die Formel 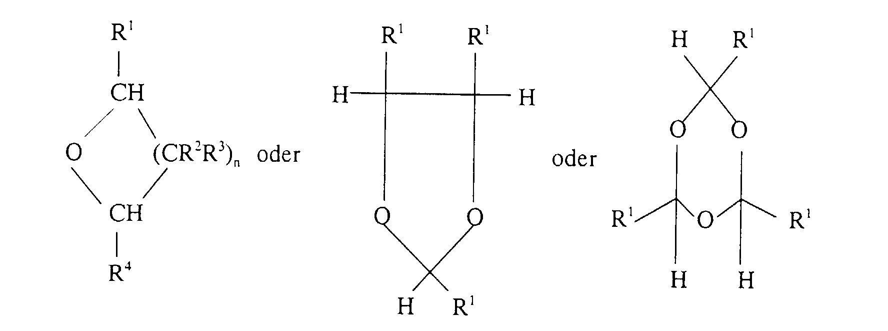 umfaßt, worin n 2 oder 4 ist und jedes R¹, R², R³ und R⁴ unabhängig für Wasserstoff oder 1 bis 20 Kohlenstoffatome enthaltendes Hydrocarbyl steht.

4. Verfahren nach Anspruch 3, in welchem R¹, R², R³ und R⁴ alle Wasserstoff sind.

5. Verfahren nach Anspruch 3, in welchem n 2 ist; und worin R¹, eines von R², beide von R³ und R⁴ Wasserstoff sind und das verbleibende R² Alkyl, das 1 - 4 Kohlenstoffatome enthält, darstellt.

6. Verfahren nach Anspruch 1, in welchem es sich bei dem Metallkation um Scandium, Yttrium, die Seltenerdmetalle, Titan, Zirkonium, Hafnium, Vanadium, Niob, Tantal, Chrom, Molybdän, Wolfram, Rhenium, Eisen, Ruthenium, Palladium, Kupfer, Gold, Zink, Zinn, Wismut oder Mischmetall handelt.

7. Verfahren nach Anspruch 6, in welchem das Metallkation Yttrium, Ytterbium, Dysprosium, Erbium, Neodym, Lanthan, Mischmetall, Scandium oder Zirkonium ist.

8. Verfahren nach Anspruch 1, in welchem der Beschleuniger eine Carbonsäure ist, deren pKa in Wasser etwa 6 oder weniger beträgt.

9. Verfahren nach Anspruch 8, in welchem die Carbonsäure aus der aus Ameisensäure, Essigsäure, Trifluoressigsäure, Acrylsäure, Methacrylsäure, Cyanoessigsäure, Nitropropionsäure und Nitrobenzoesäure bestehenden Gruppe ausgewählt ist.

## Revendications

1. Procédé de polymérisation d'éthers cycliques, comprenant la mise en contact d'un ou plusieurs oxétannes, tétrahydrofurannes, oxépannes, 1,3-dioxolannes ou 1,3,5-trioxannes, avec une zéolithe qui contient un cation métallique sélectionné dans le groupe composé du scandium, de l'yttrium, des métaux des terres rares, du titane, du zirconium, de l'hafnium, du vanadium, du niobium, du chrome, du molybdène, du tantale, du tungstène, du rhénium, du fer, du ruthénium, de l'osmium, du rhodium, de l'iridium, du palladium, du cuivre, du platine, de l'argent, de l'or, du zinc, du cadmium, du mercure, de l'aluminium, du gallium, de l'indium, du thulium, du germanium, de l'étain, du plomb, de l'arsenic, de l'antimoine, du bismuth et du mischmétal; et un accélérateur sélectionné dans le groupe composé d'anhydrides carboxyliques, d'halogénures d'acyle et d'acides carboxyliques dont le pKa dans l'eau est inférieur à environ 6.

2. Procédé suivant la revendication 1, dans lequel ledit éther cyclique est un ou plusieurs tétrahydrofurannes, oxépannes, 1,3-dioxolannes ou 1,3,5-trioxannes.

3. Procédé suivant la revendication 2, dans lequel ledit éther cyclique comprend la formule : 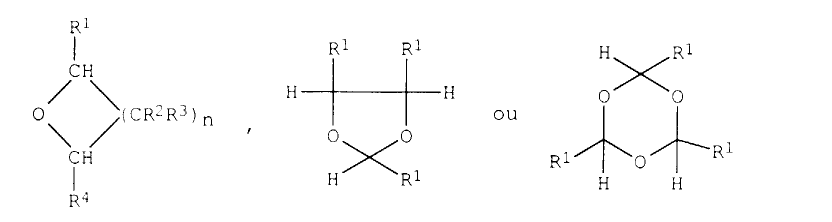 dans laquelle n est 2 ou 4, et chacun de R¹, R², R³ et R⁴ est indépendamment un hydrogène ou un hydrocarbyle contenant 1 à 20 atomes de carbone.

4. Procédé suivant la revendication 3, dans lequel R¹, R², R³ et R⁴ sont tous un hydrogène.

5. Procédé suivant la revendication 3, dans lequel n est 2 et dans lequel R¹, l'un des R², et tant R³ que R⁴ sont un hydrogène, et le R² restant est un alkyle contenant 1 à 4 atomes de carbone.

6. Procédé suivant la revendication 1, dans lequel ledit cation métallique est le scandium, l'yttrium, les métaux des terres rares, le titane, le zirconium, l'hafnium, le vanadium, le niobium, le tantale, le chrome, le molybdène, le tungstène, le rhénium, le fer, le ruthénium, le palladium, le cuivre, l'or, le zinc, l'étain, le bismuth ou le mischmétal.

7. Procédé suivant la revendication 6, dans lequel ledit cation métallique est l'yttrium, l'ytterbium, le dysprosium, l'erbium, le néodyme, le lanthane, le mischmétal, le scandium ou le zirconium.

8. Procédé suivant la revendication 1, dans lequel ledit accélérateur est un acide carboxylique dont le pKa dans l'eau est d'environ 6 ou moins.

9. Procédé suivant la revendication 8, dans lequel ledit acide carboxylique est sélectionné dans le groupe composé des acides formique, acétique, trifluoroacétique, acrylique, méthacrylique, cyanoacétique, nitropropionique et nitrobenzoïque.
